Europäisches Patentamt

European Patent Office

Office européen des brevets

(11) Publication number: **0 318 318**
**A1**

(12) **EUROPEAN PATENT APPLICATION**

(21) Application number: 88311209.6

(22) Date of filing: 25.11.88

(51) Int. Cl.⁴: **C 12 Q 1/38**

(30) Priority: 27.11.87 GB 8727794
27.11.87 GB 8727795

(43) Date of publication of application:
31.05.89 Bulletin 89/22

(84) Designated Contracting States:
CH DE FR GB IT LI

(71) Applicant: **RADIOMETER CORPORATE DEVELOPMENT LIMITED**
**The Manor Manor Royal**
**Crawley, West Sussex RH10 2PY (GB)**

(72) Inventor: **Packer, Simon David**
**1 Stuart Way East Grinstead**
**West Sussex, RH19 4RR (GB)**

**Hill, Simon Julian Roland**
**45 Sackville Gardens East Grinstead**
**West Sussex (GB)**

(74) Representative: **Jones, Helen M.M. et al**
**Gill Jennings & Every 53-64 Chancery Lane**
**London WC2A 1HN (GB)**

(54) Urine assay process and kit.

(57) Leucocyte elastase is used as a marker for white blood cells in a urine screen. To detect the presence of pyuria (more than 10 Y cells per mms) a synthetic peptide derivative substrate of elastase is contacted with a sample of urine and the products of hydrolysis are detected in the medium. The preferred substrates are chromogenic, most preferably fluorogenic. One substrate is methoxysuccinyl - alanyl-alanyl-prolyl-valyl-7-amino-4-methyl coumarin.

EP 0 318 318 A1

Bundesdruckerei Berlin

**Description**

## URINE ASSAY PROCESS AND KIT

The present invention relates to a process for assaying urine, to identify the presence of white blood cells (leucocytes) in the urine.

In hospital microbiology labs, up to 50% of samples analysed are urine sample. At least 80 or 90% of the samples tested are negative for clinically significant indications. Thorough analysis of urine samples may involve microscopy and/or culturing and is time consuming and requires skilled operators. It is therefore desirable to eliminate negative samples in order to reduce the number of samples which have to be tested by culturing and microscopy. The test must be highly specific in order that, if possible, all positive samples are identified. In order to be economically viable it must be reasonably specific in distinguishing between positive and negative samples, so that a high proportion of the negative samples are identified.

It is generally accepted that a high number of white blood cells in urine, for instance above 10 cells per $mm^3$, is clinically significant in the presence of high numbers of bacteria, for instance to detect asymptomatic infection or to distinguish between pathogens in the urine and contaminants. It can also be significant in the presence of lower counts of bacteria, since it may indicate inflammation without infection or infection by certain agents as chlamydia trachomatis, mycoplasma spp or neisseria gonorrheae. There are several methods of detecting and quantifying white blood cells in urine.

One method is by microscopy of uncentrifuged urine, either using a Gram stain or in a volumetric chamber. Microscopic evaluation can also be carried out on centrifuged urine, but centrifugation involves an extra step and is susceptible to differences in volumes used, speed and time of centrifugation and of the technicians carrying out the procedure. All microscopic methods are time consuming and require relatively skilled technicians. Furthermore it is known that the survival of leucocytes in urine is decreased by high pH, low osmolality and raised temperature.

Another recently developed method uses particle size distribution analysis using a Coulter counter, however this process uses expensive equipment and is impractical for hospital laboratories and for use on large numbers of samples.

A further recently developed test is the so-called Autotrak, marketed by Automated Microscopy System. In this automated test process a sample of urine is stained with acridine orange which stains both microoganisms and white blood cells in the sample. The stained sample is then examined using an epi-fluorescence microscope. The analysis system can differentiate between fluorescence emitted from white blood cells and from microoganisms and can therefore be used to give an indication of the number of white blood cells in the sample. One problem with this system is that it is useful solely to carry out the urine screen assay.

Chemical methods have been developed to give a quick indication of the presence of large numbers of white blood cells in urine. One such test is marketed as a dip-stick under the name Chemstrip LN, further described by Kusumi et al in JAMA 245, 1653 to 1655. The plastic strip has a filter paper and pad containing an indoxyl carboxylic acid ester that is a substrate for esterase, being converted to indoxyl which undergoes oxidation in air to form indigo which is a blue colour. Although Kusumi found the test to be sufficiently sensitive and specific as a screen for pyuria (more than 10 cells per $mm^3$) more recent reports express doubts that the test is sufficiently sensitive or specific. For instance Wilkins et al in J. Clin. Path. Ol. (1985) 38 1342 to 1345, Murray et al in J. Clin. Micro. Biol. (1987) 25 467 to 470, and Wu et al in J. Clin. Micro. Biol. (1985) 21 796 to 799 all found that the esterase strip was insufficiently sensitive and insufficiently specific to be useful in their tests. Furthermore Wilkins et al report that the colour change was sometimes difficult to interpret. Wu et al reported that the use of some antibiotics, in particular the amino glycosides can have an inhibitory effect on leucocyte esterase activity.

In EP-A-0237394 chromogenic substrates of cholinesterase are used in an assay for determining the presence of leucocytes in urine.

In EP-A-0012957 (and US-A-4278763) chromogenic indoxyl ester derivatives of amino acids and peptides are disclosed as substrates for esterases in neutrophilic leucocyte granulocytes. They are apparently hydrolysed by proteolytic enzymes including elastase, chymotrypsin and trypsin for detection of those enzymes in aqueous solutions and in body fluids other than urine.

In EP-A-0158225 (and iiS 47585081 substrates which are esters of phenols are used in an assay for esterolytic or proteolytic enzymes in leucocytes for instance in urine. One substrate is an alanyl ester of a phenol derivative.

Certain other urine screens do react with white blood cells. For example the Bac-T-Screen (Marion Scientific Laboratory) system is a colorimetric filtration system in which urine is passed through a filter and then safranin dye is applied to the filter. Both bacteria and leucocytes in the urine retain the dye to produce a pink coloration. However, the test cannot distinguish between bacteria and white blood cells and furthermore a high proportion of urines are not analysable by this method since various types of debris in the urine, including pigments and blood, block the screen to prevent passage of the sample.

A further automated test uses bioluminescence, utilising the ability of fire-fly luciferase to react quantatively with bacterial ATP to give a luminescence output. The system is described by Ulrich and Wannlund in American Clinical Product Review, November 1984. The system detects some mammalian ATP from white blood cells, as well as bacterial ATP, but cannot distinguish between the two. Furthermore the system is relatively labour intensive and is expensive in the consumable reagents utilised.

It is known that leucocytes are rich in protease activity, partly due to the presence of elastase. Barrett in Methods in Enzymology, (1981) 80 581 to 588 states that the enzyme may be purified from white blood cells from blood or may be isolated from purulent sputum as well as human spleen and rheumatoid synovial membrane.

Elastase has been implicated as being responsible for diseases such as pulmonary emphysema, probably causing proteolysis of lung tissue which is a characteristic of that disease. It has also been implicated in other inflammatory diseases, in the production of mediators of inflammation itself. The assay of the enzyme is of importance in investigations of such inflammatory diseases and in the search for treatments.

In Analytical Biochemistry 99, 53-64 (1979) Castillo et al assessed a variety of synthetic peptide substrates to assay for elastase. The various substrates comprise the same peptide group, but differed in the leaving group released on the hydrolysis by elastase. One of the leaving groups formed p-nitroaniline. Two of the substrates yielded fluorescent compound, 1-methoxy-3-naphthylamine and 7-amino-4-methyl-coumarin, which can be detected fluorometrically. Another substrate was a thiobenzyl ester, yielding benzyl mercaptan which was reacted with a further reagent, either with 5, 5′-dithiobis (2)-nitrobenzoic acid) (Ellman's reagent) to produce 3-carboxy-4-nitrothiophenoxide anion, or with 4, 4′- dithiodipyridine to produce 4-thiopyridone, which products can be detected by measuring the absorption of light of 412nm and 324nm, respectively. Another substrate was the ethyl ester of the peptide, hydrolysis of which by elastase produces ethanol which can identified by including in the assay mixture NAD- and liver alcohol dehydroganase, which will produce NADH + which can be analysed by measuring the absorption at 340nm. All of the assays were capable of detecting small amounts of elastase in reaction mixtures. The elastases used were purified.

In "Methods of Enzymatic Analysis" 3rd edition, volume 5 pages 176-184, Tschesche et al describe the assay for human leucocyte elastase using synthetic chromogenic or fluorogenic peptide substrates, including Suc-Ala-Ala-Ala-NA, MeOSuc-Ala-Ala-Pro-Val-NA and Suc-Ala-Ala-Val-NA. The method described uses extracted enzyme and the only specific description of the extraction process is from human blood.

In Hoppe-Seyler's Z. Physiol. Chem. (1980) 361 1413-1416 Wenzel et al compare the kinetics of the action of purified human leucocyte elastase and porcine pancreatic elastase on Suc-Ala-Ala-Val-NA, MeOSuc-Ala-Ala-Pro-Val-NA and Ac-Ala-Ala-Pro-val-NA.

A further method of assaying for elastase as been described in B.B.R.C. 75, 194 - 199 (1977), where Ashe and Zimmerman use elastin as a substrate and identify the product by fluorescence detection. The elastase enzymes tested were purified.

It is known that elastase in plasma is complexed with an inhibitor, known as alpha$_1$-protease inhibitor, which completely inhibits the enzymatic activity of the elastase. It is also known that some elastase in plasma is bound to alpha$_2$-macroglobulin. The enzyme in the latter complex retains some enzymatic activity. Indeed, in Nature (1977) 267, 61-63 Twumasi et al suggest that human leucocyte elastase is activated towards Suc (Ala)$_3$ NA by human $\alpha_2$-macroglobulin. However, because there is relatively little of that complex and because it is eliminated quickly from the circulation, its identification is of little value.

One way around this problem has been described in J. Clin. Chem. Clin. Biochem., 22 (1984), 693 - 697 where Neumann et al describe an immuno assay for the inhibitor-elastase complex. The process described suffers several disadvantages, including the fact that it is a multi-step process and thus time consuming and labour-intensive, as well as being expensive in reagents. we have found that inhibition of elastase activity occurs not only in plasma, but also in other bodily fluids, such as in urine. Part of the inhibition of elastase in urine may be due to the presence of blood in urine, which occurs in a proportion of the population, especially those with certain diseases.

Other substances are known to inhibit alastase activity and have been investigated for possible use as therapeutic agents. Ashe and Zimmerman (op cit.) describe the inhibition of human granulocyte elastase by cis-unsaturated fatty acids, such as oleic acid. Vered et al in Am. Rev. Respir. Dis (1985), 131 - 133 describe the inhibition of elastase by a peptide derived from streptococcus pneumoniae, which can be dissociated from the elastase by mild denaturants, e.g. by sodium dodecyl sulphate.

Castillo et al (op cit) state that DMSO dimethyl sulphoxide) has been shown to stimulate elastase, although the reason for this is not elucidated. Bieth and Wermuth (op cit), found that DMSO as well as DMF (dimethylformamide) had inhibitory effects on elastase, however further experiments are described in J. Biol. Chem. (1980) 255, 9289-9294 where Lestienne ad Bieth describe the activation of leucocyte elastase by excess substrate, hydrophobic solvents and ionic strength. In J. Biol. Chem. (1981) 256 10256-10258 Boudier et al describe the activation of leucocyte elastase by cathepsin G, which is another leucocyte protease. The effect is not seen with synthetic substrates and so is probably due to the proteolytic action of cathepsin G on elastin. All these experiments were conducted on purified elastase. In JP-A-57-166982 the activation of a precursor of pancreatic elastase by treatment with an acidic protease for instance produced by Rhizopus over a 10-hour period is described.

According to the invention a new process for assaying urine to detect the presence of white blood cells comprises forming an assay mixture containing a sample of urine and an enzyme substrate, which is capable of being hydrolysed by elastase in the assay mixture to form a compound whose presence can be detected in the assay mixture, and then detecting the compound.

The sample on which the assay is carried out may be a direct urine sample or maybe diluted, or may be derived from urine by centrifugation. In order to avoid extra steps in the procedure, it is preferred to use a direct urine sample.

In the process the substrate is preferably a very specific and sensitive leucocyte elastase substrate and is substantially incapable of being hydrolysed by leucocyte esterase, trypsin or chymotrypsin, i.e. it is hydrolysed slowly by such proteases relative to its rate of hydrolysis by elastase or not at all.

Suitable substrates are compounds of the formula I

$R^1-A^4-A^3-A^2-A^1-CONH-R^2$

in which $A^1$ is an amino acid residue having a non-polar side chain, preferably selected from alanyl valyl, phenylalanyl and methionyl, $A^2$ is a bond or an amino acid residue selected having a non-polar side chain, preferably selected from prolyl, alanyl and leucyl, $A^3$ is a bond or is an amino acid having a non-polar side chain, preferably an alanyl residue and $A^4$ is a bond or is an amino acid residue having a non-polar side chain, preferably an alanyl residue, $R^1$ is glutaryl or is an N-terminal blocking group and $R^2$ is selected such that the compound $R^2NH_2$ can be detected in the assay medium. Preferably $R^1$ is selected from acetyl(Ac), succinyl(Suc), methoxysuccinyl(MeOSuc), t-butyloxycarbonyl (Boc) and glutaryl(Glt). Preferred compounds are tri- or tetrapeptide derivatives eg compounds in which $A^1$ is alanyl or valyl, preferably valyl, and $A^2$ is alanyl or prolyl and $A^3$ is alanyl. MeOSuc is a particularly preferred group $R^1$.

The elastase substrate used in the assay is a peptide derivative which is specific for the elastase enzyme. There are many known peptide derivatives which are suitable as substrates, and these include amide, or less preferably ester, derivatives of:

Ac - Ala - Ala - Ala
Ac - Ala - Ala - Pro - Ala
Ac - Ala - Ala - Pro - Phe
Ac - Ala - Pro - Ala
Boc - Ala
Boc - Ala - Ala
Boc - Ala - Ala - Ala
Boc - Ala - Ala - Pro - Ala
Boc - Ala - Ala - Pro - Val
Glt - Ala - Ala - Ala

MeOSuc - Ala - Ala - Pro - Val
Suc - Ala - Ala - Ala
Suc - Ala - Ala - Pro - Met
Suc - Ala - Ala - Val
Suc - Ala - Try - Leu - Val
Pyr - Pro - Val

Preferred substrates are blocked derivatives of Ala-Ala-Pro-Val and Ala-Ala-Val, preferably amide derivatives.

The most preferred substrate is an amide derivative of MeOSuc - Ala - Ala - Pro - Val, which is described in Castillo et al, in Analytical Biochemistry (1979) 99, 53-64.

Detection of the compound in the assay mixture is most conveniently carried out by optical detection methods, e.g. turbidimetric, spectrophotometric or, most preferably, fluorimetric methods. The derivatising group of the peptidyl substrate is such that when the substrate is hydrolysed by elastase the compound produced is directly detectable by optical means or, less preferably, indirectly detectable e.g. it may be reacted further within the sample to produce a product wich is detectable by optical means.

The optical detection means may comprise means for detecting fluorescence, absorption or reflection of visible light of particular wavelengths or by turbidity changes. Thus for instance the compound produced by hydrolysis of the substrate may be one whose fluorescence properties are different to that of the substrate itself, which may fluorescence at a different wavelength or not at all. Alternatively the compound may be a different colour to that of the suhstrate and the absorption or reflection of light can be measured spectrophotometrically or simply visually.

The compound which is produced by the hydrolysis reaction may alternatively not be directly observable by the optical detection means but may be reacted with other components of the reagent mixture to produce a compound which can be observed. The further reaction may be a simple chemical reaction or may be an enzyme-catalysed reaction. For instance the compound may be coupled with a coupling agent to produce a dye, e.g. an axo dye. The compound or a further reaction product of the compound may affect the optical properties of another component of the assay mixture e.g. by absorbing fluorescence emitted by a component, for instance, as described in US4495293.

Preferably the derivatising group is a Fluorogenic group. Examples of such suitable substrates are beta-naphthylamides, including derivatives of 4-methoxy-beta-naphthylamide (4 MβNA) and beta-naphthylamide (βNA), coumarin derivatives such as 7-amino-coumarin derivatives, e.g. 7-amino-4-methyl coumarin (AMC) derivatives and 7-amino-4-trifluoromethyl coumarin (AFC) derivatives. The fluorescent compounds produced by the hydrolysis of such substrates can be detected by known methods and apparatus.

Another suitable derivatising group is one which produces a coloured compound whose presence can be observed directly, either visually or spectrophotometrically by measuring absorption of light, usually of a specific wavelength. The substrate can be, for instance an amide derivative of p-nitroaniline or p-diethylaminoaniline or an ester of p-nitro-phenol as well an ester of 2-naphthol.

Other derivatising groups are ones which yield compounds not directly observable but which can be further reacted with other components present in the reagent mixture to produce optically detectable compounds. These additional components may be specifically added to the mixture or may be inherently present in the sample. The substrate may for instance be a thiobenzylester or an ethyl ester whose hydrolysis products can be detected by methods described in Castillo et al (op. cit.).

Another substrate which can be used is elastin provided with an optically detectable label as described by Quinn and Blout in BBRC(1970) 40, 328-333. Elastin is less preferred since it is insoluble in water.

The most preferred substrate for use in the invention is MeOSuc - Ala - Ala - Pro - Val - AMC which is highly specific for human leucocyte elastase and whose hydrolysis product AMC can be deleted in minute amounts by fluorescence detection.

The hydrolysis of the substrate can be continuously monitored by observing the presence or otherwise of the compound in the assay mixture at intervals. Alternatively the end point only of the reaction may be observed or the assay mixture may be analysed after predetermined period of time, not necessarily after all the substrate has been hydrolysed. The assay process may be used to determine the number of white blood cells in the sample or simply to indicate that the number of cells is above a cut-off value preferably above 8 or 10 cells/mm$^3$ urine.

We have found that in some urine samples there may be inhibitors of elastase activity and it is therefore convenient to add reagents to the assay mixture which reduce or eliminate this inhibition. We have found that there are certain protease enzymes which can reduce the inhibition without effecting the activity of the enzyme itself or having a proteolytic effect on the enzyme substrate. It is preferred to incorporate one of those enzymes into the assay mixture.

We have found that incorporating such an enzyme into an assay mixture is of value in reducing inhibition in the test of body fluids other than urine. According to anyother aspect of the invention, therefore, a new process for assaying a sample of body fluid for elastase comprises forming an assay mixture containing:

    a) a liquid sample,

    b) an elastase substrate, which is capable of being cleaved by elastase to yield a compound whose presence in the mixture can be detected, and

    c) a protease, which is capable of reducing elastase inhibition by elastase inhibitor but incapable of hydrolysing said elastase substrate, and then analysing the mixture to detect the presence of the said compound.

This aspect of the invention is based on the surprising discovery that certain protease enzymes are specifically able to reduce the inhibition of leucocyte elastase activity, without adversely effecting the elastase enzyme itself and without hydrolysing the elastase substrate, either to release the compound whose presence can be detected (i.e. to produce false positive results), or to change the composition of the substrate so that it no longer acts as an efficient substrate for elastase (i.e. to produce or maintain false negative results).

The sample which is treated in the second aspect of the invention is derived from a biological fluid, either from a human or another animal, for instance from blood, fluid discharge from sites of inflammation or other wounds, sputum or urine. The sample used in the assay may be the fluid itself or may be derived from it by various purification processes, for instance it may comprise a preparation of whole or lysed white blood cells or a protein extract thereof.

Protease enzymes which have the desired inhibition-reducing activity (and which do not hydrolyse MeOSuc - Ala - Ala - Pro - Val - AMC) include: lysing enzymes, protease Type XVIII from Rhizopus species, protease Type IV from Streptomyces caespitosus, protease Type IX from Bacillus polymixa and Protease Type IV from Bacillus polymixa Protease enzymes which we have found to have hydrolytic activity on the elastase substrate MeOSuc - Ala - Ala - Pro - Val - AMC or which do not satisfactorily reduce inhibition and are therefore not useful as ingredients of the assay mixture include Protease type IXI from Aspergillus susae, Protease Type XIII from Aspergillus saitoi, Protease from Aspergillus caryzae, Protease type III from Papaya, protease type I from Bovine pancreas, Protease type XXIV (Bacterial) and Protease from Subtilisin Carlsberg. All these proteins are available from Sigma.

The preferred protease enzymes for use in the present inventions are Lysing Enzymes and Protease type XVIII from Rhizopus species.

We have found that the inclusion of further components in the assay mixture can have additional inhibition reducing (or activating) properties on the elastase activity towards the substrate.

Further components suitable as activators should possess some surface active properties and/or protein denaturing activities, although should not have significant deleterious effects on the elastase or protease. Suitable further activating components for including in the mixture are saponin, Triton and CHAPS (3-Cholamidopropyl)-dimethylammonio) -1-propanesulfonate). The assay mixture also usually contains buffers and can contain metal ions, stabilisers, etc.

The substrate is suitably present in the assay mixture at a concentration in the range 0.02%w/v to 2%w/v preferably 0.05% to 0.5%, for instance about 0.2%. These values may sometimes be higher, for instance, if spectrophotometric detection is used. Any protease has been found to be useful at concentrations in the range 0.05%w/v to 5%w/v preferably 0.2% to 2%, for instance about 0.5%w/v. If the additional activator is to be included in the mixture it is generally present at a concentration in the range 0.25 to 10%ow/v, preferably 1 to 5%ow/v, for instance about 2.5%.

The assay process may be carried out by adding the reagents separately to the container in which the reaction is to be carried out, either as dried compounds or in aqueous solution. Preferably, however, at least some of the reagents and preferably all the reagents are provided in preprepared form, preferably in a container, either in optionally frozen solution form, or, preferably, in dried form, more preferably as a film on an internal surface of the container. The apparatus for carrying out the process may be in the form of an absorbent substrate e.g. a pad or paper carrying the reagents, in the interstices of which the assay mixture can be contained.

The reagents are preferably provided in a form such that they do not require a separate reconstitution step e.g. to adequately dissolve the reagent, before addition of the urine sample to be assayed. Thus in the preferred process the liquid component is derived entirely from the urine sample. The urine sample is usually in the range 5 to 250µl, preferably 10 to 100µl

According to the invention a kit suitable for carrying out the process of the invention comprises an assay container, that is suitable for containing the assay mixture, and elastase substrate. Preferably the substrate is provided in the container, usually in dried form. In the second aspect of the invention and in the preferred embodiment of the first aspect the kit comprises also protease of the above defined type. Preferably the substrate and/or any protease is provided in the form of the same or different dried films optionally comprising a water-soluble film-forming stabiliser on a surface of the same or different container.

A preferred form of kit comprises a plate having a plurality of wells (which act as containers), e.g. a microtitre plate. Such apparatus is well known for carrying out biochemical assays which are followed by visual observation of colour changes, by measurement of absorption of light and by fluorescence detection. Another form of kit comprises an absorbent material in the interstices of which the liquid assay mixture can be contained and which thus provides the container. The absorbent material, which can be formed of e.g. paper or a pad, is preferably mounted on a strip of non-absorbent insoluble material, e.g. a plastics material, for ease of handling. The absorbent material can be prepared so that it contains some or all the necessary reagents.

Usually the kit also comprises instructions for its use for testing urine.

Where there is more than one reagent the reagents may be provided in the same or in separate containers. In general, it is preferred to provide the reagents all in a single container so that by addition of the sample to be assayed to the container the entire assay mixture is provided without additional manipulations (i.e. transferring materials from one container to another) being necessary.

The reagents are provided in amounts suitable to provide the above-mentioned concentrations in the reagent mixture. In a microtitre plate the sample size used is generally in the range 5 to 250µl more usually 10 to 100µl and so the substrate is provided in an amount in the range about 10µg to 500µg, for instance about 100µg and the protease is provided in an amount in the range of about 25 to 1250µg, for instance about 250µg.

Suitably the assay for elastase in urine is carried out in conjunction with an assay for bacteriuria, carried out on the same urine. Such further method should be a non-cultural method, preferably giving a result within the same time scale as the elastase assay. Conveniently it uses the same detection means and is preferably carried out on the same microtitre plate, in a separate well from the elastase test.

Suitable tests for detection of bacteriuria are for instance the nitrate test (Griess test), the catalase test, described in J. Lab. and Clin. Med. (1961) 490-494, glucose oxidase test, a test using fluorogenic or chloro-unorgenic substrates that are hydroysable by bacteria, for instance as described in EP-A-91837 or, preferably, a limulus amoebocyte lysate assay, described by Jorgensen et al (1973) Applied Micro. Biol. 26 38-42 to detect endotoxin produced by gram negative bacteria, preferably using a fluorogenic or chromogenic sbstrate hydrolysable by an enzyme produced in the gelation cascade, that is known per se.

We have found that the results of the elastase assay gives a more reliable indication of pyuria than the esterase test of Chemstrip LN and is therefore more useful in a urine screen since it will be less likely to give false results. The following examples illustrate the invention:

EXAMPLE 1

A reagent solution was made up containing 380ml of a 5% phosphate buffer at pH of 7.5 20ml of a 15% w/v solution of PVA made up in distilled and deionised water, 12 g of Saponin (available from Sigma), a solution of 1 g of fluorogenic elastase substrate (MeOSuc-Ala-Ala-Pro-Val-AMC) in 100ml of 2 Me (2-methoxyethanol, BDH Analar) 50 µl of the resultant solution was dosed into the wells of a microtitre plate. The solutions were dried by vacuum drying at a raised temperature, under conditions such that the solution neither boiled nor froze, until substantially all the solvent had been removed.

The prepared plates were then used to detect the presence of white blood cells in urine samples At the same time a number of peptidase enzymes were tested for their properties in reducing inhibition of elastase activity. Where such peptidase enzymes were used they were added in an amount of 0.25mg. per well. Fresh urine samples are dosed into each well at 50µl per wall. The plate is then incubated at 37°C for 30 minutes.

Reading Results

The wells are read in a fluorimeter. Using a zenon lamp the well is excited with light peaking at 360mm wavelength.

Immediately emission from the well is read by counting the emissions peaking at 450mm wavelength using a photomultiplier tube.

Each of the urine samples was investigated by microscopy to find the actual number of white blood cells and red blood cells present in the urine sample. The numbers of red blood cells are observed merely to discover if they contribute to the inhibition of elastase activity. The microscopy results are shown in Table I.

Results of over 900 counts are considered positive for this assay.

TABLE I

| Urine Sample No. | MICROSCOPY RESULT | |
| --- | --- | --- |
| | White Blood Cells ($\mu l^{-1}$) | Red Blood Cells ($\mu l^{-1}$) |
| 8 | Nil | Nil |
| 27 | 80 | 1 |
| 30 | 50 | > 1000 |
| 51 | 200 | 1000 |

The results of the florescence detection experiments are shown in Table II. Counts of over 900 are positive for this assay.

TABLE II

| Urine Sample | Control (no peptidase) | Rhizopus peptidase | Aspergillus Saitoi peptidase | Aspergillus oryzde peptidase | |
| --- | --- | --- | --- | --- | --- |
| No 8 | 610 | 588 | 561 | os* | |
| | 572 | 604 | 557 | os | |
| | 533 | 657 | 483 | os | |
| No 27 | 2192 | 2146 | 1854 | os | |
| | 2325 | 2085 | 1855 | os | |
| | 2435 | 2054 | 1716 | os | |
| No 30 | 202 | 1285 | 277 | | 2557 |
| | 273 | 1483 | 401 | | 2267 |
| | 239 | 1316 | 286 | | 2287 |
| No 51 | 677 | 2956 | 1243 | os | |
| | 767 | 2796 | 1275 | os | |
| | 724 | 3103 | 1232 | os | |
| Water Control | 596 | 578 | 700 | os | |
| | 544 | 577 | 547 | os | |
| | 657 | 604 | 562 | os | |

*os - off scale

The results show that the test for elastase activity shows positive results in urine sample number 27, which contains clinically significant numbers of white blood cells as shown by microscopy. The results also show that the assay results are negative for the urine sample which contains no white blood cells (urine sample no. 8). For urine samples no.'s 30 and 51, the results of assays containing no peptidase show that the elastase is inhibited in the urine sample. By selecting an appropriate enzyme the inhibition can be reduced. Thus the rhizopus peptidase seems to destroy inhibition in both urine samples. The aspergillus saitoi peptidase affects the inhibition of urine sample no. 51 but not urine sample no. 30. Aspergillus oryzae peptidase hydrolyses the substrate itself and so gives false positive results.

EXAMPLE 2

Example 1 was repeated in the absence of any added protease and in the presence (1.2mg per well) and absence of saponin on five other urine samples. The results are shown in Tables III and IV. Fluorimeter counts of over 900 are positive in this assay.

7

TABLE III

| | Microscopy result | |
|---|---|---|
| | White blood cells ($\mu l^{-1}$) | Red blood cells ($\mu l^{-1}$) |
| 21 | 60 | 0 |
| 29 | 450 | 800 |
| 33 | 70 | 0 |
| 58 | 500 | 0 |
| 38 | 0 | 0 |

TABLE IV

| URINE SAMPLE | CONTROL (NO SAPONIN) | WITH SAPONIN |
|---|---|---|
| 21 | 1152, 1629, 1076 | 1758, 1664, 1257 |
| 29 | 1152, 1069, 1076 | os, 3829, os |
| 33 | os, os, 3898 | 2728, 3282, 3032 |
| 58 | 987, 956, 877 | os, 4064, os |
| 38 | 493, 468 | 544, 535 |

The results indicate that urines 21, 29 and 58 show an improved positive response to the assay with saponin present. Urine 33 is an example of an unaffected result. The result of the assay of urine 38 (true negative) shows that the assay itself is not effected by saponin.

Example 3

A buffer solution was made up using 36.15g disodium hydrogen orthophosphate (anhydrous), 4.0g potassium dihydrogen orthophosphate and 9.0g CHAPS in 800 ml water (distilled and deionised). The solution was made up to 980 ml with water. A solution of 2 g the same substrate used in the previous examples was made up in 20 ml DMSO and added to the buffer. 5.0g Rhizopus peptidase was dissolved in the substrate solution. 50 $\mu l$ portions of the solution was dosed into the wells of a microtitre plate and the solutions dried by vacuum drying. The plates were used in the manner as in Example 1 to give similar results.

**Claims**

1. A process for assaying urine to detect the presence of white blood cells comprising forming an assay mixture containing a sample of urine and an enzyme substrate, which is capable of being hydrolysed by elastase in the assay mixture to form a compound whose presence can be detected in the assay mixture, and then detecting the compound.

2. A process according to claim 1 in which the urine sample is a direct urine sample.

3. A process according to claim 1 or 2 in wich the elastase substrate is substantially incapable of being hydrolysed by leucocyte esterase, trypsin or chymotrypsin.

4. A process according to any preceding claim in which the elastase substrate is a compound of the formula I

$R^1$-$A^4$-$A^3$-$A^2$-$A^1$-CONH-$R^2$

in which $A^1$ is an amino acid residue selected from alanyl, valyl, phenylalanyl and methionyl, $A^2$ in a bond or an amino acid residue selected from prolyl, alanyl and leucyl, $A^3$ is a bond or is an alanyl residue and $A^4$ is a bond or is an alanyl residue, $R^1$ is glutaryl or is an N-terminal blocking group and $R^2$ is selected such that the compound $R^2NH_2$ can be detected in the assay medium.

5. A process according to claim 4 in which $R^1$ is selected from acetyl(Ac), succinyl(Suc), methoxysuccinyl(MeOSuc), t-butyloxycarbonyl (Boc) and glutaryl(Glt).

6. A process according to claim 4 or 5 in which the substrate is a tri-or tetrapeptide derivative,

preferably in which A[1] is alanyl or valyl, preferably valyl, and A[2] is alanyl or prolyl and A[3] is alanyl.

7. A process according to claim 1 or claim 2 in which the elastase substrate is a peptidyl derivative selected from

Ac - Ala - Ala - Ala
Ac - Ala - Ala - Pro - Ala
Ac - Ala - Ala - Pro - Phe
Ac - Ala - Pro - Ala
Boc - Ala
Boc - Ala - Ala
Boc - Ala - Ala - Ala
Boc - Ala - Ala - Pro - Ala
Boc - Ala - Ala - Pro - Val
Glt - Ala - Ala - Ala

MeOSuc - Ala - Ala - Pro - Val
Suc - Ala - Ala - Ala
Suc - Ala - Ala - Pro - Met
Suc - Ala - Ala - Val
Suc - Ala - Try - Leu - Val and
Pyr - Pro - Val
preferably, a derivative of MeOSuc - Ala - Ala - Pro -Val.

8. A process according to any preceding claim in which detection of the compound in the assay mixture is carried by an optical detection method, preferably selected from turbidimetric, spectrophotometric or, most preferably fluorimetric methods.

9. A process according to claim 4 in which the compound is directly detectable by said optical detection means, preferably having different fluorescent properties to the substrate, and more preferably being a coumarin derivative.

10. A process according to any preceding claim in which the substrate is MeOSuc - Ala - Ala - Pro - Val - AMC.

11. A process according to any preceding claim in which the assay mixture also contains a protease enzyme which is capable of reducing or eliminating elastase inhibition, but is incapable of hydrolysing the substrate.

12. A process according to any preceding claim in which the assay mixture also contains an activator which has elastase-inhibition-reducing properties, preferably a surface active material, more preferably selected from saponin, triton and CHAPS.

13. A process according to any preceding claim in which the assay mixture is formed by adding the urine sample to an assay container which already contains some or all of the other reagents for forming the assay mixture, preferably in dried form.

14. A kit suitable for carrying out a process according to any preceding claim, comprising an assay container, that is suitable for containing the assay mixture, and elastase substrate, and optionally a protease enzyme as defined in claim 11 and/or an activator as defined in claim 12.

15. A kit according to claim 10 in which the substrate is provided in the container, preferably in dry form.

16. A kit according to claims 14 and 15 in which the kit is provided with instructions for its use for testing urine.

17. A kit according to any of claims 14 to 15, which also comprises reagents for assaying another sample of said urine for bacteriuria and a container therefor, and which preferably comprises a plate having a plurality of wells, preferably a microtitre plate.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | ANALYTICAL BIOCHEMISTRY, vol. 99, no. 1, 1979, pages 53-64, Academic Press Inc., San Diego, CA., US; M.J. CASTILLO et al.: "Sensitive substrates for human leukocyte and porcine pancreatic elastase: A study of the merits of various chromophoric and fluorogenic leaving groups in assays·for serine proteases"<br>* Pages 53-54; page 60, column 1, lines 28-48 *<br>--- | 1,4,5,7,10 | C 12 Q    1/38 |
| D,X | CHEMICAL ABSTRACTS, vol. 81, no. 11, 16th September 1974, page 176, no. 59948r, Columbus, Ohio, US; E. KASAFIREK et al.: "Significance of the N-acyl residue of L-alanyl-L-alanyl-L-alanine p-nitroanilide for cleavage for pancreatic elastase", & FEBS (FED. EUR. BIOCHEM. SOC.) LETT. 1974, 40(2), 353-6<br>* Abstract *<br>--- | 1,4,7 | |
| D,X | CHEMICAL ABSTRACTS, vol. 78, no. 17, 30th April 1973, page 162, no. 107421a, Columbus, Ohio, US; G. FEINSTEIN et al.: "N-acetyl-(L-Ala)3-p-nitroanilide as a new chromogenic substrate for elastase", & BIOCHEM. BIOPHYS. RES. COMMUN. 1973, 50(4), 1020-6<br>* Abstract *<br>---                                -/- | 1,4,7,8 | TECHNICAL FIELDS SEARCHED (Int. Cl.4)<br><br>C 12 Q<br>C 12 N |

The present search report has been drawn up for all claims

| Place of search | Date of completion of the search | Examiner |
|---|---|---|
| THE HAGUE | 23-12-1988 | MEYLAERTS H. |

**European Patent Office**

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 103, no. 15, 14th October 1985, page 771, no. 123907d, Columbus, Ohio, US; Z. TETERE et al.: "Enzyme substrates. I. Synthesis of N-substituted di- and trialanines", & LATV. PSR ZINAT. AKAD. VESTIS, KIM. SER. 1985, (3), 338-41 * Abstract * | 1,4,5,7 | |
| D,X | CHEMICAL ABSTRACTS, vol. 93, no. 25, 22nd December 1980, page 343, no. 233722x, Columbus, Ohio, US; H.R. WENZEL et al.: "Synthesis and analytical use of 3-carboxypropionyl-alanyl-alanyl-valine-4-nitroanilide: a specific substrate for human leukocyte elastase", & HOPPE-SEYLER'S Z. PHYSIOL. CHEM. 1980, 361(9), 1413-16 * Abstract * | 1,4,3,7 | |
| D,X | H.U. BERGMEYER: "Method of enzymatic analysis", 3rd edition, vol. 5, chapter 2, 1984, pages 176-184, Verlag Chemie, Weinheim, DE; H. TSCHESCHE et al.: "Leukocyte elastase" * Pages 177-178 * | 1,4-8 | TECHNICAL FIELDS SEARCHED (Int. Cl.4) |
| D,A | EP-A-0 237 394  (ARLEY-GUILLAUBEY LABORATORIES) * Page 2 * | 1 | |
| A | PATENT ABSTRACTS OF JAPAN, vol. 7, no. 7 (C-144)[1152], 12th January 1983; & JP-A-57 166 982 (AMANO SEIYAKU K.K.) 14-10-1982 * Abstract * | | |

The present search report has been drawn up for all claims

| Place of search | Date of completion of the search | Examiner |
|---|---|---|
| THE HAGUE | 23-12-1988 | MEYLAERTS H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................

& : member of the same patent family, corresponding document

EPO FORM 1503 03.82 (P0401)